# EUROPEAN PATENT APPLICATION

(11) **EP 1 555 321 A1**
(43) Date of publication of application: **20.07.2005**
(21) Application number: 04290107.4
(22) Date of filing: 15.01.2004
(51) Int. Cl.: C12N 15/54, C12N 9/12, C12N 15/82, A01H 5/00, A01H 17/00

(54) **CCaMK involved in nodulation and endomycorrhization**

(71) Applicant: INSTITUT NATIONAL DE LA RECHERCHE AGRONOMIQUE, 75007 Paris (FR)
(72) Inventor: Debelle, Frédéric, 31400 Toulouse (FR); Levy, Julien, 3100 Toulouse (FR); Bres, Cécile, 84170 Monteux (FR); Rosenberg, Charles, 31320 Castanet Tolosan (FR)
(74) Representative: Vialle-Presles, Marie José

(57) **Abstract**

The invention relates to new Ca²⁺-calmodulin-dependent protein kinases (CCaMKs) from legumes

The invention also relates to the use of CCaMKs for regulating nodulation and/or endomycorrhization in plants.

## Description

The invention relates to polypeptides and polynucleotides involved in nodulation and mycorrhization in plants.

A wide range of terrestrial plants are able to associate, through their root system, with microbial symbionts.

The best known of these endosymbiotic systems are those occurring between soil bacteria collectively named rhizobia and roots of many legume (family Leguminosae) species. The legume-rhizobia symbiosis fixes as much nitrogen worldwide as the chemical fertilizer industry due to the ability of rhizobial bacteria to induce the morphogenesis of a new plant organ, the legume root nodule, in which they fix nitrogen.

Another important type of symbiotic relationships between plants and microorganisms is the arbuscular mycorrhizae (AM) symbiosis, that occurs not only in legumes but in most land plants (a notable exception is the Brassicaceae family which includes *Arabidopsis thaliana)* and involves fungi of the order *Glomales* (GIANINAZZI-PEARSON, *Plant Cell,* vol. 8, p.1871, 1996; HARRISON, *Annu. Rev. Plant Physiol. Plant Mol. Biol.,* vol. 50, p.361, 1999 ; BRUNDRETT, *New phytologist,* vol. 154, p.275, 2002).

Although the legume-rhizobia symbiosis and the AM symbiosis may at first sight appear not to have much in common, both the fungal and bacterial partners possess the highly restricted ability to trigger a plant host genetic program permitting controlled and localized infection.

Studies on the legume-rhizobia symbiosis have led to the identification of the structure and importance of rhizobial signals, the lipochito-oligosaccharidic Nod factors, which initiate symbiotic responses on the roots of legume hosts and are required for recognition, controlled infection and nodule formation (DÉNARIÉ, *et al., Annu Rev Biochem,* vol.65, p.503, 1996; LEROUGE *et al., Nature,* vol.344, p.781, 1990; GEURTS_and BISSELING, *Plant Cell,* vol.14, Suppl, S239, 2002). At pico-nano molar concentrations these molecules induce a variety of responses including rapid calcium influx and calcium spiking, specific gene induction, alterations in epidermal cell morphology and cortical cell mitosis (GEURTS and BISSELING, 2002, précité).
Genetic studies in the model legumes *Medicago truncatula* and *Lotus japonicus* have led to the identification of genes involved in the perception and transduction of Nod factors. Recently genes coding for two types of transmembrane receptor-like serine/threonine kinases with putative extracellular regions containing LysM domains (LysM-RLKs), required for Nod factor responses and rhizobial infection, have been cloned ( RADUTOIU *et al., Nature,* vol.425, p.585, 2003; LIMPENS *et al., Science,* vol.302, p.630, 2003; MADSEN *et al.,* *Nature,* vol.425, p.637, 2003). The gene products are hypothesized to form heterodimeric Nod factor receptors (RADUTOIU *et al.,* 2003, précité).

Downstream of these genes, three genes have been mapped in *M truncatula, DMI1, DMI2* and *DMI3* that are required for both nodulation and the formation of arbuscular mycorrhizae (the AM symbiosis). Mutants in *DMI1, DMI2* and *DMI3* are blocked for most responses to Nod factors: They have a similar phenotype and do not exhibit induction of root hair branching (but instead form hair swelling), early nodulin gene expression and cortical cell division (CATOIRA *et al., Plant Cell,* vol.12, p.1647, 2000). However, they differ in their ability to respond to Nod factors by the induction of sharp oscillations of concentration of cytoplasmic calcium (calcium spiking), which is lost in *dmi1* and *dmi2* but not in *dmi3* mutants (EHRHARDT *et al., Cell,* vol.85, p.673, 1996; WAIS *et al., Proc Natl Acad Sci U S A,* vol.97, p.13407, 2000). Furthermore, mutants in *DMI1* and *DMI2 (= NORK)* genes, which are unable to form nodules and mycorrhizae (CATOIRA *et al.,* 2000, précité), are defective for most responses to Nod factors, including the calcium spiking response, but are still able to generate the rapid calcium influx response (EHRHARDT *et al.,* 1996, précité; SHAW and LONG, *Plant Physiol,* vol.131, p.976, 2003).

Recently, *DMI2* was shown to encode a LRR receptor-like protein kinase (NORK; ENDRE *et al., Nature,* vol.417, p.962, 2002).

The inventors have now isolated and characterized the *DMI3* gene of *M. truncatula,* and have found that it encodes a member of the small plant family of chimeric Ca²⁺-calmodulin-dependent protein kinases. They have also identified the orthologous *DMI3* gene in pea.

Members of the CCaMK group have been described in a number of plants ranging from the moss *Physcomitrella* to higher plants, both monocotyledonous and dicotyledonous. DMI3 is highly similar to rice, tobacco, and lily CCaMKs (73.5 % identity between *Medicago* and lily CCaMK). All these proteins share with the more common plant calcium-dependent protein kinases (CDPK), an N-terminal kinase domain. However they differs from CDPKs by the structure of their C-terminal calcium-binding regulatory domain that is more similar to a mammalian visinin-like domain (with three calcium-binding EF hands) than to the calmodulin-like domain typical of CDPKs (with four EF hands). Between the kinase and calcium-binding domains lies a calmodulin-binding domain that overlaps an autoregulatory domain. This structure allows regulation of the kinase activity by both calcium and calmodulin (SATHYANARAYANAN *et al., J Biol Chem,* vol.276, p.32940, 2001; TAKEZAWA *et al., J Biol Chem,* vol.271, p.8126, 1996).

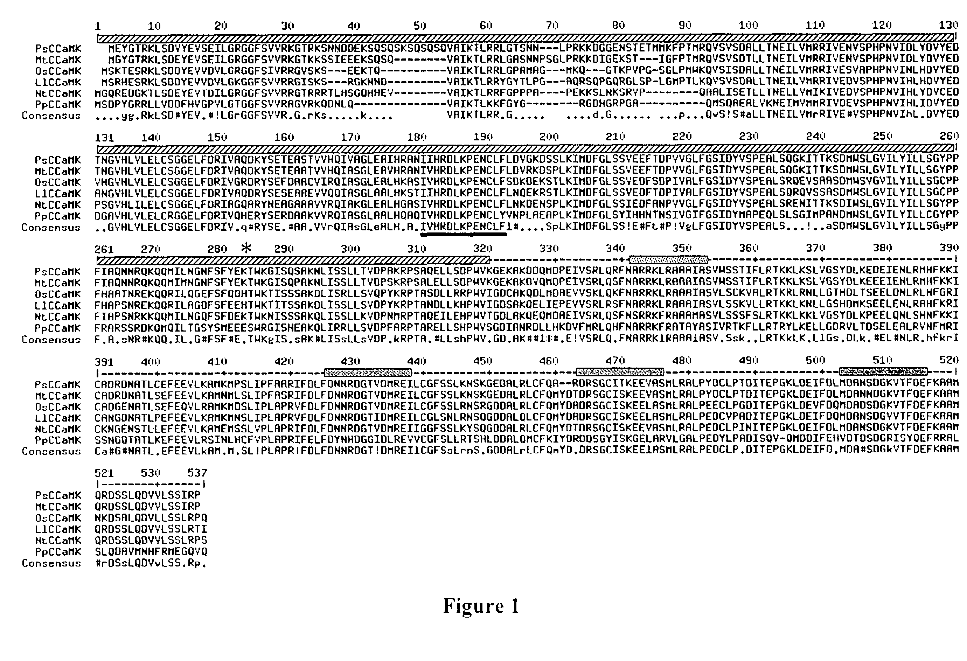

Figure 1 shows the comparison of CCaMKs of *M. truncatula* (MtCCaMK) and *P. sativum* (PsCCaMK) characterized by the inventors to previously characterized CCaMKs from lily (LICCaMK : U24188), rice (OsCCaMK : AK070533), tobacco (NtCCaMK : AF087813) and *Physcomitrella* (PpCCaMK : AY155462). The serine/threonine kinase domain ( 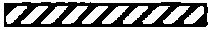 ), calmodulin-binding site ( 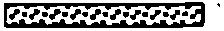 ), calcium-binding EF hand motifs ( 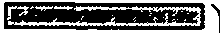 ) and autophosphorylation site (*) of CCaMKs are indicated above the sequences. The kinase active site signature ( 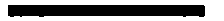 ) is indicated under the sequences. A putative bipartite nuclear localization signal can be detected between positions 60 and 75 in *M. truncatula* CCaMK sequence, but it is not present in the *P. sativum* sequence.

Little is known about the biological role of CCaMKs in plants. The fact that the lily, tobacco and rice CCaMKs are preferentially expressed in developing anthers and root tips (POOVAIAH *et al., Planta,* vol.209, p.161, 1999; WANG and POOVAIAH, *J Biol Chem,* vol.274, p.12001, 1999) has led to the suggestion that they could play a role in mitosis and meiosis (YANG and POOVAIAH, *Trends Plant Sci,* vol. 8, p.505, 2003).

The inventors show here for the first time, through the use of *DMI3* mutants, that a CCaMK plays a role in the signaling pathway leading to endomycorrhization and in the developmental process of nodulation in legumes. *DMI3* appears to act immediately downstream of calcium spiking and upstream of all other symbiotic responses. Without being bound to any particular mechanism, it may be hypothesised that the calcium spiking, and not the rapid calcium influx, is the calcium signature recognized by DMI3, the role of which would be to translate this signal to various cellular components controlling nodulation and mycorrhization responses.

The present invention provides means to regulate the nodulation and/or endomycorrhization responses of plants to microbial symbionts.

In particular, the invention provides a Ca²⁺-calmodulin-dependent protein kinase (CCaMK) comprising an amino acid sequence having at least 80% identity or at least 90% similarity, preferably at least 85% identity or at least 90% similarity, more preferably at least 90% identity or at least 95% similarity, and still more preferably at least 95% identity or at least 99% similarity, with the polypeptide SEQ ID NO: 2

Unless otherwise specified, the sequence identity or similarity values provided herein are calculated using the BLAST programs (ALTSCHUL *et al., Nucleic Acids Res.,* vol.25, p.3389, 1997) under default parameters, and the whole length of the reference sequence as a comparison window.

According to a preferred embodiment of a CCaMK of the invention, it is selected among the *Medicago truncatula* CCaMK of SEQ ID NO: 2 and the *Pisum sativum* CCaMK of SEQ ID NO: 4.

The present invention also provides chimeric polypeptides comprising a CCaMK of the invention.

The polypeptides according to the invention can be prepared by any conventional method known in the art, for example by chemical synthesis. They can also be prepared by culturing an host cell containing an expression vector comprising a polynucleotide encoding said polypeptide, under conditions suitable for the expression of the polypeptide, and recovering the polypeptide from the host cell culture.

They can further be purified by any of the means known in the art. Various methods of protein purification are described, e.g., in Guide to Protein Purification, ed. Deutscher, Meth. Enzymol. 185, Academic Press, San Diego, 1990; and SCOPES, Protein Purification: Principles and Practice, Springer Verlag, New York, 1982.

The invention also provides an isolated polynucleotide encoding a CCaMK polypeptide of the invention, as well as the complement of said polynucleotide.

A ''polynucleotide encoding a CCaMK" is any polynucleotide comprising the information allowing the translation into said polypeptide. It may consist of an isolated coding sequence (CDS); it may also consist of a whole length mRNA comprising said CDS, or of the corresponding cDNA or genomic DNA.

Examples of polynucleotides encoding a CCaMK of the invention are represented by SEQ ID NO: 1, which is a genomic DNA sequence encoding the *Medicago truncatula* CCaMK of SEQ ID NO: 2, and by SEQ ID NO: 3, which is a genomic DNA sequence encoding the the *Pisum sativum* CCaMK of SEQ ID NO: 4.

Other polynucleotides encoding a CCaMK polypeptide of of the invention can be obtained by methods well known in the art, such as screening genomic or cDNA libraries from any leguminous plant species able to form root nodules with rhizobia, using nucleic acid probes and/or primers obtained from SEQ ID NO: 1, 3 or degenerate probes or primers obtained from SEQ ID NO: 2 or 4.

By way of example, polynucleotides of the invention can be obtained from genomic or cDNA libraries of Papilionoideae, in particular those belonging to any of the genus *Pisum, Glycine, Phaseolus,* or *Medicago.*

The invention also includes specific sub-fragments of a polynucleotide of the invention, as well as nucleic acid fragments selectively hybridising therewith. Preferably these fragments comprise at least 10, preferably at least 15, more preferably at least 20, still more preferably at least 50 consecutive nucleotides of a polynucleotide of the invention.

"Specific fragments" refers to nucleic acid fragments having a sequence that is found only in the nucleic acids sequences encoding a CCaMK polypeptide of the invention, and is not found in nucleic acids sequences encoding related polypeptides of the prior art. This excludes the nucleic acid fragments that consist of a sequence shared with any of the previously known CCaMK genes.

"Selectively hybridising fragments" refers to nucleic acid fragments which can hybridise, under stringent conditions, with nucleic acid sequences encoding a polypeptide of the invention, without hybridising with nucleic acid sequences encoding related polypeptides of the prior art. This excludes the nucleic acid fragments that cross hybridize under stringent conditions with any of the known CCaMK genes

"Stringent conditions" refers to hybridization conditions under which a nucleic acid will detectably hybridize to its target sequence, but not to other sequences in a mixture of nucleic acids. It is well known in the art that nucleic-acid hybridization is affected by such conditions as salt concentration, temperature, or organic solvents, in addition to the base composition, length of the complementary strands, and the number of mismatched bases between the hybridizing nucleic acids.

Generally, stringent conditions are selected to be about 5-10 °C. lower than the thermal melting point (Tₘ) for the duplex formed by a given sequence and its complement in a hybridization medium of a defined ionic strength and pH. By comparison, low stringency conditions are generally selected to be about 15-30°C below the Tₘ.

Polynucleotides of the invention as well as fragments thereof may be used to prepare probes or primers for detecting and/or amplifying a nucleic acid sequence encoding a polypeptide of the invention.

They may eventually be labeled, for instance with a radioactive element (³²P, ³⁵S, ³H, ¹²⁵I) or by a non-isotopic molecule (for example, biotin, acetylaminofluorene, digoxigenin, 5-bromo-desoxyuridin, fluorescin).

Methods for preparing and using probes and primers are described, for example, in SAMBROOK *et al.,* Molecular Cloning: A Laboratory Manual, 2nd ed., vol. 1-3, ed. Sambrook et al., Cold Spring Harbor Laboratory Press, Cold Spring Harbor, NY, 1989; Current Protocols in Molecular Biology, ed. Ausubel et al., Greene Publishing and Wiley-Interscience, New York, 1987 (with periodic updates); and INNIS *et al.,* PCR Protocols: A Guide to Methods and Applications, Academic Press: San Diego, 1990.

Said probes and primers are also part of the invention. In particular, the invention encompasses any set of primers comprising at least one primer consisting of a specific fragment of a polynucleotide of the invention.

The present invention also provides expression cassettes comprising a polynucleotide encoding a CCaMK of the invention, under control of regulatory sequences which will direct the transcription and translation of the polynucleotide in an intended host cell.

Typically, said regulatory sequences comprise at least a promoter that drives expression in the host cell. They generally also comprise a transcription initiation start site, a ribosome binding site, an RNA processing signal, a transcription termination site, and/or a polyadenylation signal.

All or part of said regulatory sequences can be derived from the natural CCaMK gene, or consist of heterologous regulatory sequences.

By way of example, one may use the endogenous promoter of the CCaMK gene eventually altered in order to up-regulate or down-regulate gene expression; one may also replace the endogenous CCaMK promoter by an heterologous promoter providing a stronger or weaker level of expression, and/or a different expression profile (i.e a promoter conferring inducible or constitutive, environmentally or developmentally-regulated, or cell or tissue-specific/selective expression).

A large choice of promoters suitable for expression in varied hosts is available in the art.

In particular, promoters capable to direct transcription in plant cells include a large variety of promoters that can be obtained for instance from plants, plant viruses, or bacteria such as *Agrobacterium.* They include constitutive promoters, i.e. promoters which are active in most tissues and cells and under most environmental conditions, tissue or cell specific promoters which are active only or mainly in certain tissues or certain cell types, and inducible promoters that are activated by physical or chemical stimuli.

Non-limitative examples of promoters that are commonly used in plant cells are the cauliflower mosaic virus (CaMV) 35S promoter, the Nos promoter, the rubisco promoter. Advantageously, one may use a root-specific or root-preferred promoter.

Non limitative examples of root-specific or root-preferred promoters include the maize allothionein promoter (de FRAMOND et al., FEBS 290, 103-106, 1991); EP 452269), the acid chitinase promoter described by SAMAC et al., (Plant Physiol. 93, 907-914, 1990), the soybean root-specific glutamine synthetase promoter described by HIRE et al. (Plant Mol. Biol. 20, 207-218, 1992), or the root specific promoters described in WO/9113992 or US 5,837,848.

The invention also provides a recombinant vector resulting from the insertion of a polynucleotide or an expression cassette of the invention into a host vector.

The recombinant vectors of the invention can be prepared by the conventional techniques of genetic engineering. The host vector may be any vector suitable for transfection of an intended host cell. By way of non-limitative example, vectors suitable for transfection of plant cells and/or for the establishment of transgenic plants can be derived from the tumor-inducing (Ti) plasmid *of Agrobacterium tumefaciens.*

Vectors of the invention are preferably expression vectors, wherein a polynucleotide of the invention encoding a CCaMK polypeptide is placed under control of appropriate regulatory sequences.

The vectors of the invention may also comprise one or several marker gene(s) which confers a selectable phenotype on the host cells. Example of marker genes are reporter genes such as genes coding for beta-glucuronidase (GUS), for green fluorescent protein (GFP), for luciferase. Other examples of marker genes are antibiotic resistance genes, such as resistance to kanamycin, G418, bleomycin, hygromycin, or herbicide resistance genes, such as resistance to Basta.

The present invention also comprises:
- prokaryotic or eukaryotic host cells, transformed by a polynucleotide of the invention.
   Preferably, said host cells are plant cells;
- plant tissues or organs, or tissue cultures comprising transformed plant cells of the invention;

Introduction of a polynucleotide of the invention into host cells or tissues can be effected by means well known in the art, for instance by transfection, microinjection, electroporation, transduction, ballistic introduction, or the like. In the case of plant cells, one can also use *Agrobacterium rhizogenes* or advantageously, *Agrobacterium tumefaciens* mediated transformation.

The transformed cells, tissues, or organs of the invention can be used to regenerate transgenic plants. Many methods of cell transformation and plant regeneration for numerous plant species are known in the art.

The invention also encompasses transgenic plants, genetically transformed by a polynucleotide of the invention.

Preferably, the polynucleotide is stably integrated within the genome of the plant and is passed on to successive generations. Descendants of the initial transgenic plants are thus also part of the invention, provided that they remain genetically modified by a polynucleotide of the invention.

The invention also comprises parts from the plants of the invention such as flowers, seeds, leaves, branches, fruit, provided that these parts comprise cells genetically modified by a polynucleotide of the invention.

The invention also provides a method for regulating nodulation and/or endomycorrhization in a plant wherein said method comprises modulating the expression or activity of a CCaMK in said plant.

According to a preferred embodiment of the invention, said CCaMK is a CCaMK of the invention, as defined above.

However, the findings of the present invention allow to presume that previously known CCaMKs from non-leguminous plants, whose function was unknown until now, also play a part in endomycorrhization in said plants, and thus that modulating the expression or activity of said CCaMKs may allow to regulate endomycorrhization.

According to the invention, the expression of a CCaMK in a plant can be increased or decreased for the purpose of modulating nodulation and/or endomycorrhization.

The expression of a CCaMK in a plant can be increased, for instance, by expressing in said plant a DNA construct producing said polypeptide, or by altering the endogenous promoter of the *CCaMK* gene in order to up-regulate gene expression; conversely, a decrease of the expression of a CCaMK can be obtained for instance by expressing in said plant a polynucleotide inducing the silencing of a *CCaMK* gene, for instance by antisense inhibition or cosuppression. It is also possible to use ribozymes targeting CCaMK mRNA, or to alter the *CCaMK* promoter in order to down-regulate gene expression.

The CCaMK activity in a plant can be increased, for instance by expressing a mutant CCaMK devoid of the autoinhibitory domain located around the calmodulin binding domain, such as the mutant disclosed by RAMACHANDIRAN et al. (J. Biochem. (Tokyo), 121, 984-90, 1997). Conversely, the CCaMK activity in a plant can be decreased, for instance, by expressing a CCaMK having a mutation impairing the function of the kinase domain.

The invention also provides means for evaluating the nodulation and/or endomycorrhization characteristics of a plant, in particular a legume.

In particular, the invention provides:
- a method for identifying an allele of a *CCaMK* gene associated with given nodulation and/or endomycorrhization characteristics, wherein said method comprises isolating a nucleic acid fragment comprising the *CCaMK* gene or a portion thereof from at least one plant expressing said phenotype and sequencing said fragment;

The invention further provides:
- a method for identifying a polymorphism in the *CCaMK* gene associated with nodulation and/or endomycorrhization phenotype, wherein said method comprises identifying, as described above, at least two different alleles of *CCaMK* associated with different nodulation and/or endomycorrhization phenotypes and comparing the sequences of said alleles.

Once a polymorphism has been identified, reagents and kits allowing the routine detection of said polymorphism can be designed. Commonly used reagents are nucleic acid probes, or restriction enzymes, or PCR primers, or combinations thereof. The choice of a reagent or of a combination of reagents depends of the nature of the polymorphism.

Preferred kits and reagents are those comprising a set of primers allowing specific PCR amplification of a DNA segment spanning the polymorphic locus. For microsatellites and insertion/deletion polymorphisms, PCR primers may be sufficient, since the allelic forms of the polymorphism may be differentiated by the size of the amplification product. In the case of single nucleotide polymorphisms (SNP), one will generally also use a restriction enzyme, allowing to differentiate the allelic forms by the presence or size of restriction fragments.

The invention also provides a method for testing a plant for its nodulation and/or endomycorrhization characteristics, wherein said method comprises detecting whether an allele of a *CCaMK* gene associated with given nodulation and/or endomycorrhization characteristics is present in said plant.

According to a preferred embodiment of the method of the invention, said nodulation and/or endomycorrhization characteristics are the lack of nodulation and/or endomycorrhization, or a reduced nodulation and/or endomycorrhization and the allele of the *CCaMK* gene associated with said phenotype expresses no CCaMK, or a lower level of CCaMK, or a less active CCaMK than the wild type allele, or an inactive CCaMK.

Examples of alleles resulting in said phenotype are:
- alleles having a deletion of all or part of the *CCaMK* gene, or that are splicing variants of the *CCaMK* gene, and result in the expression of a truncated CCaMK;
- alleles having a mutation resulting in the expression of a less active or inactive variant of CCaMK ;
- alleles having a mutation in the *CCaMK* promoter, resulting in a lower expression of CCaMK:

By way of example, some alleles of *Medicago truncatula* and *Pisum sativum* CCaMKs found by the inventors to be associated with said phenotype in are listed in Table I below:

**TABLE 1**

| Genetic background | Mutation |
|---|---|
| *Medicago truncatula* | 14 bp deletion: 198-203/stop |
| | CGA/TGA : R97/stop |
| *Pisum sativum* | CAA/TAA: Q230/stop |
| | CAA/TAA : Q230/stop |
| | CAA/TAA : Q230/stop |
| | TGG/TGA : W240/stop |
| | TGT/TGA : S224/stop |
| | CTT/C-T : L188/stop |
| | GGG/AGG: G202/R |
| | TCT/TTT : S24/F |

All these mutations are located in the kinase domain.

The present invention will be further illustrated by the additional description which follows, which refers to examples of obtention and use of polynucleotides encoding a CCaMK of the invention. It should be understood however that these examples are given only by way of illustration of the invention and do not constitute in any way a limitation thereof.

### EXAMPLE 1: CLONING AND CHARACTERIZATION OF M. TRUNCATULA DMI3.

The *DMI3* gene was amplified from genomic DNA of *M. truncatula* wild-type Jemalong A17 (CATOIRA *et al.,* 2000, précité) and of two *dmi3* mutants, TRV25 (SAGAN *et al., in Biological nitrogen fixation for the 21st century* Elmerich, Kondorosi, Newton, Ed, Kluwer Academic Publishers, p.317-318, 1998) and T1-5, both defective for nodulation. T1-5 mutant was obtained from a population of EMS mutagenized seeds, deriving from Jemalong A17 *ENODII-GUS* (JOURNET *et al., Mol Plant Microbe Interact,* vol.14, p.737, 2001), by screening seedlings for the lack of GUS activity in roots after treatment with Nod factors.

The PCR primers used for amplification are listed in table II below:

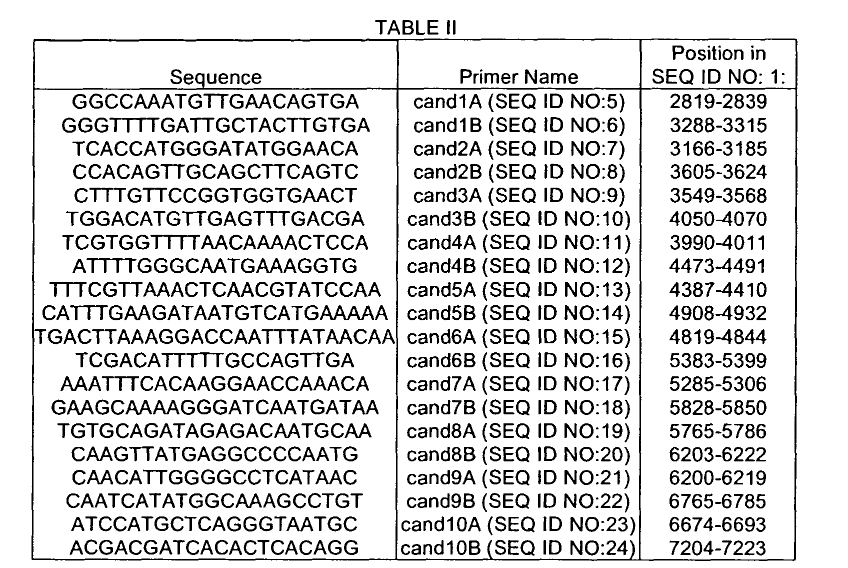

PCR amplification were performed using the Taq polymerase (IN VITROGEN) according to the manufacturer instructions (MgCl2 2,5 mmol/l).

PCR program:
* 1 cycle (94° for 5 min)
* 30 cycles (94° for 15 s, 55° for 15 s, 72° for 1 min)
* 1 cycle (72° for 7 min).

The genomic sequence of the wild-type *DM13* is represented in the annexed sequence listing under SEQ ID NO: 1.

The *DMI3* gene comprises 7 exons. The full length open reading frame of 1,569 nucleotides codes for a protein of 523 amino-acids with a predicted molecular mass of 58,600 Da.

The position of the introns and the exons in the sequence SEQ ID NO: 1 is as follows:
exon1: 3171-3901
exon2: 4467-4521
exon3: 4600-4710
exon4 : 5000-5232
exon5 : 5764-5971
exon6 : 6627-6686
exon7 : 6769-6942

Analysis of the deduced polypeptide sequence indicated that DMI3 belongs to the calcium and calmodulin-dependent protein kinase group of serine-threonine protein kinases, together with proteins encoded by genes previously characterized from tobacco, lily, rice and the moss *Physcomitrella* (Figure 1).

Like these proteins, DMI3 shares with the more common plant calcium-dependent protein kinases (CDPK), an N-terminal kinase domain. However it differs from CDPKs by the structure of its C-terminal calcium-binding regulatory domain that is more similar to a mammalian visinin-like domain (with three calcium-binding EF hands) than to the calmodulin-like domain typical of CDPKs (with four EF hands). Between the kinase and calcium-binding domains lies a calmodulin-binding domain that overlaps an autoregulatory domain. This structure allows regulation of the kinase activity by both calcium and calmodulin.

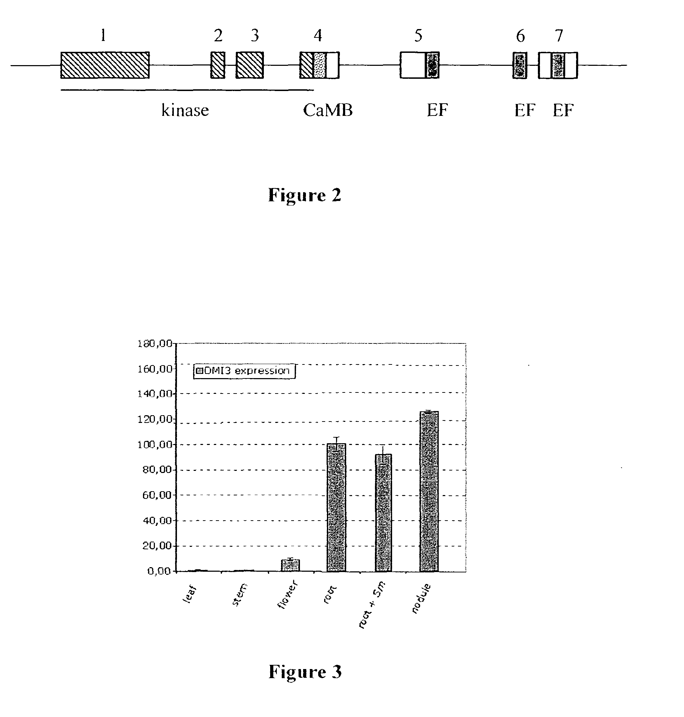

The intron-exon structure of the *DMI3* gene and the position of the predicted protein domains and motifs are schematized in Figure 2. The kinase domain ( 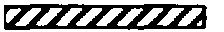 ), calmodulin binding (CaMB) domain ( 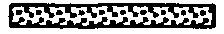 ) and calcium-binding EF hands ( 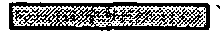 ) are indicated.

### EXAMPLE 2: FUNCTIONAL COMPLEMENTATION OF A DMI3 MUTATION BY THE WILD-TYPE CCAMK CLONE.

Roots of seedlings of the TRV25 *dmi3* mutant, defective for nodulation (SAGAN *et al.,* 1998, précité), were transformed with *Agrobacterium rhizogenes* strain Arqual harboring the pF5 plasmid which carries the wild-type *DMI3* gene.

A 11 kb *XbaI* fragment, carrying the wild type *DMI3* coding sequence and 1,040 bp upstream of the start codon, was cloned into pCAMBIA2201 (www.cambia.org). The resulting pF5 recombinant plasmid was introduced into *A. rhizogenes* Arqual by electroporation (MCCORMAC *et al., Molecular Biotechnology,* vol.9, p.155, 1998). Hairy root transformation of TRV25 *dmi3* mutant was carried out as described (BOISSON-DERNIER *et al., Mol Plant Microbe Interact,* vol.14, p.695, 2001), with the following modifications. Transgenic roots were first selected on Fahraeus agar medium supplemented with kanamycin (20 mg 1-1), then the transformed seedlings were transferred to pouches and inoculated with the rhizobial symbiont *Sinorhizobium meliloti* GMI6526 = RCR2011 (pXLGD4), which carries a *lacZ* reporter gene to facilitate the *in planta* visualization of the bacteria.

Nodules were scored two weeks after inoculation. Experiments were performed twice. 90 % of plants exhibited transformed roots as detected by GUS activity. In total, 45 plants transformed by pF5 were tested, 36 of them nodulated with an average of 5 nodules per plant. A X-Gal treatment and microscopic examination of a representative sample of nodules revealed the presence of bacteria inside the nodules. No nodule could be detected on 26 control plants transformed by the pCAMBIA2201 vector.

This complementation together with the genetic evidence described in Example 1, demonstrate that the *DMI3* encodes a CCaMK required for nodulation and mycorrhization phenotype.

### EXAMPLE 3: EXPRESSION PATTERN OF DMI3 MRNA IN M. TRUNCATULA.

*DMI3* mRNA levels were measured by quantitative RT-PCR in leaves, stems, flowers, roots, roots 48h post-inoculation with S. *meliloti,* and root-nodules of *M truncatula,* and normalized against *MtACTIN2 (MtACT2)* that is constitutively expressed in all tissues tested.

Total RNA was extracted and treated by DNAseI. cDNA was synthesized from 1 µg total RNA using the Taqman Gold RT-PCR kit (Perkin-Elmer Applied Biosystems) in a total volume of 50 µl. Quantitative RT-PCR reactions were performed in triplicate on 6.5 µl cDNA using the SYBR-GreenR PCR Master kit (Perkin-Elmer Applied Biosystems) (40 cycles of 95°C for 10 s, 58°C for 1 min) and real-time detection was performed on the ABI 7700 and analyzed using the GeneAmp 5700 SDS software (Perkin-Elmer Applied Biosystems).

Primers used:
DMI3-1: TCATTGATCCCTTTTGCTTCTCGT (SEQ ID NO:25),
DMI3-2: GATGCTACTTCCTCTTTGCTGATGC (SEQ ID NO:26),
MtACT2: TGGCATCACTCAGTACCTTTCAACAG (SEQ ID NO:27),
MtACT2-2: ACCCAAAGCATCAAATAATAAGTCAACC (SEQ ID NO:28).

The results are shown in Figure 3. A 10-fold higher expression of *DMI3* was observed in roots compared to flowers, whereas no significant expression was detected in leaves or stems. Further, a slight upregulation of the root expression of *DMI3* in roots as well as in root nodules, in response to the symbiotic partner S. *meliloti,* could be observed 48 h post inoculation.

The *DMI2 (=NORK)* gene, another *M. truncatula* gene required for nodulation and mycorrhization, whose mutation results in a symbiotic phenotype similar to that of the *dmi3* mutant TRV25, apart from calcium spiking, was studied in parallel. Its pattern of expression was similar to that of *DMI3,* although the level of expression was higher for *DMI2* (data not shown).

### EXAMPLE 3 4: CLONING AND ISOLATION OF A DMI3 HOMOLOGUE IN PEA.

Mutants with phenotypes and syntenic map positions similar to that of TRV25 have been described in pea. As *dmi3* mutants, pea mutants at the *sym9* locus are altered immediately downstream of the calcium spiking response induced by treatment of root hairs by Nod factors, and are defective for root hair curling, infection thread formation, nodulation, and endomycorrhization.

PCR amplification was performed from genomic DNA of wild-type and *svm9* mutants *of P. sativum.* The plants are listed in Table III below:

**Table III**

| Variety | | Reference |
|---|---|---|
| Frisson | Wild type | (DUC and MESSAGER, *Plant Science,* vol.60, p.207, 1989) |
| P1 | EMS *sym9* mutant of Frisson | ( DUC and MESSAGER, 1989, précité) |
| P2 | EMS *sym9* mutant of Frisson | ( DUC and MESSAGER, 1989, précité) |
| P3 | EMS *sym9* mutant of Frisson | ( DUC and MESSAGER, 1989, précité) |
| P53 | EMS *sym9* mutant of Frisson | ( SAGAN et *al., Plant Science,* vol.100, p.59, 1994) |

The primers used, designed on the basis of the *M truncatula DMI3* sequence, are listed in Table IV below:

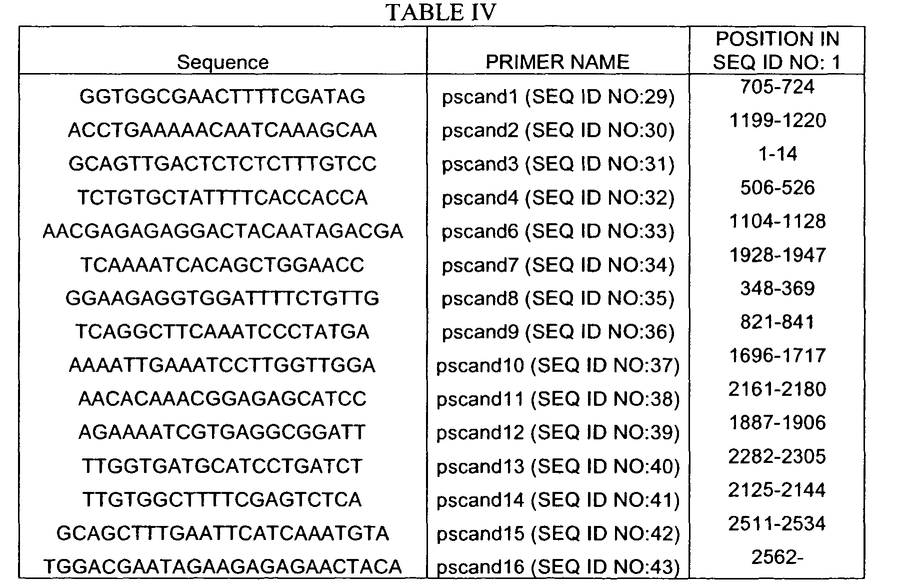

PCR amplification were performed using the Taq polymerase (IN VITROGEN) according to the manufacturer instructions (MgCl2 2,5 mmol/l).

PCR program:
* cycle (94° for 5 min)
* 30 cycles (94° for 15 s, 55° for 15 s, 72° for 1 min)
* 1 cycle (72° for 7 min).

The genomic sequence obtained from wild-type *P. sativum* cv Frisson is represented in the annexed sequence listing under SEQ ID NO: 3.

The gene comprises 7 exons. It encodes a CCaMK with high overall similarity (90 % identity) to that of *M. truncatula.*

The position of the introns and the exons in the sequence SEQ ID NO: 2 is as follows:
exon1: 294-1048
exon2: 1219-1273
exon3: 1349-1459
exon4: 1541-1773
exon5: 1978-2192
exon6: 2284-2330
exon7: 2416-stop

Each sequenced CCaMK from eight *sym9* mutants of P. *sativum,* obtained in three different genetic backgrounds, exhibited single base-pair alterations in the kinase domain predicted to alter the CCaMK function. This further supports a role of CCaMK in nodulation and mycorrhization. In addition, it allows the molecular characterization of a major gene controlling both nodulation and mycorrhization in pea, an important grain legume crop.

Various combinations of the following primers designed on the basis of the *M. truncatula DMI3* sequence were used for PCR amplification of *P. sativum SYM9* genomic DNA in the different wild-type cultivars and *sym9* mutants.

## Claims

1. An isolated Ca²⁺-calmodulin-dependent protein kinase (CCaMK) comprising an amino acid sequence having at least 80% identity or at least 90% similarity, with the polypeptide SEQ ID NO: 2.

2. A CCaMK of claim 1, selected among the *Medicago truncatula* CCaMK of SEQ ID NO: 2 and the *Pisum sativum* CCaMK of SEQ ID NO: 4.

3. An isolated polynucleotide selected among:
a) a polynucleotide encoding a CCaMK according to any of claims 1 or 2;
b) the complement of polynucleotide a) ;
c) a polynucleotide which hybridizes under high stringency conditions with a polynucleotide a) or b);
d) a fragment of at least 15 consecutive nucleotides of a polynucleotide a) or b) above.

4. An isolated polynucleotide of claim 3, wherein said polynucleotide comprises a coding sequence having at least 80% identity, with the coding sequence of the polynucleotide SEQ ID NO: 1, or with the coding sequence of the polynucleotide SEQ ID NO: 3.

5. An expression cassette comprising a polynucleotide encoding a CCaMK according to any of claims 1 or 2.

6. A recombinant vector comprising a polynucleotide encoding a CCaMK according to any of claims 1 or 2.

7. A host cell transformed by a polynucleotide encoding a CCaMK according to any of claims 1 or 2.

8. A host cell according to claim 7, wherein said cell is a plant cell.

9. A transgenic plant genetically modified by a polynucleotide encoding a CCaMK according to any of claims 1 or 2.

10. A method for regulating nodulation and/or endomycorrhization in a plant wherein said method comprises modulating the expression or activity of a CCaMK in said plant.

11. The method of claim 10, wherein said CCaMK is a CCaMK of any of claims 1 or 2.

12. The method of any of claims 10 or 11, wherein the expression or activity of said CCaMK in said plant is increased for enhancing nodulation and/or endomycorrhization.

13. The method of any of claims 10 or 11, wherein the expression or activity of said CCaMK in said plant is decreased for reducing nodulation and/or endomycorrhization.

14. A method for identifying an allele of a *CCaMK* gene associated with given nodulation and/or endomycorrhization characteristics, wherein said method comprises isolating a nucleic acid fragment comprising the *CCaMK* gene or a portion thereof, from at least one plant expressing said phenotype and sequencing said fragment.

15. A method for testing a plant for its nodulation and/or endomycorrhization characteristics, wherein said method comprises detecting whether an allele of a *CCaMK* gene associated with given nodulation and/or endomycorrhization characteristics is present in said plant.


SEQUENCE LISTING

<110>  INRA

<120>  CCaMK involved in nodulation and endomycorrhization

<130>  MJPbv539/122

<160>  43

<170>  PatentIn version 3.1

<210>  1
<211>  11200
<212>  DNA
<213>  Medicago truncatula

<220>
<221>  exon
<222>  (3171)..(3901)

<223>
<220>
<221>  exon
<222>  (4467)..(4521)

<223>
<220>
<221>  exon
<222>  (4600)..(4710)

<223>
<220>
<221>  exon
<222>  (5000)..(5232)

<223>

<220>
<221> exon
<222> (5764)..(5971)
<223>

<220>
<221> exon
<222> (6627)..(6686)
<223>

<220>
<221> exon
<222> (6769)..(6942)
<223>

<400> 1
ggcacagccg ttacacatgg ggtcgggtgt ttcaatggag attccgtttg cagatagtca 60
tatggcattg gatacgcaga tgcctggaag aaaaaggagc actccggagg atatggtgga 120
gaagactgtt gagagaaggc agaaaaggat gatcaagaat cgggaatctg ctgcccgttc 180
aagagcacgg aaacaggtga tctctctctt tctttatttc aagtgttgta actcgcaagt 240
ccttattatt tgattggttt ttgttgtaat gaatacccta aacctgaata ataactttaa 300
caagcaggct tacacaaatg aattggagat taaagtttca cgcctggaag aggaaaatga 360
aatgctgagg aaacgaaagg tttgattctt ctttgaaatt ataagtttaa ttgatttgac 420
tgagtatgtt tttataatga tcctaattcc tttcattccc cagattgagt agtttgtaca 480
tttgtggatt ataatttcag cataagatag gagacagtct cagtttcaat tactatacaa 540
atctctgcat ttttttggat taatgaaact ctgcatatta tgttctctag ttgagacctg 600
agcatgtgtc atttgcattt gttaaatttg aattaaagag gaatcggtcc attgatcaga 660
agcaagtctt gtaacgtaga ataaacattg ttaaatcatt acaccatcag cattgccgtc 720
ttgatgttat ttatttggat gtatctctct tatggcctct gctctatctt ttacatagtc 780
attaagaatt gttcaggaaa ataaatcaca tgagacacat ttaattttgt gattgacaaa 840
cacaaatgaa catgttagtt ctgatgcaac ttttggatta ttctgctctg aatataccac 900
aatgaatctc actaagggga tttaaatttt tccattcata caaaaacctt accgtgtgac 960
atgtttccca cttagagcac agagttgcac ctttgaaata aaaattactg ctttcattaa 1020
ttgagtttca tatcaggaac tatatgcctt gaatttccac tgcttcgtta agtcagtttt 1080
ttttatattg ggtgatttgg aatttgattt atcggctcaa cagtctttgc tctttttcat 1140
tcaattagca gtgcttgtgg aagatcttgg atcgtacaac catacatgct ataataaaaa 1200
ctatatctat atgcacattg tatccatggt catttttacc tgttaaaatc atgggaacaa 1260

aattgctctt ttatacatgc taacttactc ctttgccttg tatatactat cagattatat 1320
tattaatttt tgtcctcaac atgcaggagc tagagaatat gttgccttgt gcaccgattg 1380
ctgaacctaa atatcagcta cgccgaatag catcctgtcc attctgagat ctttgtacct 1440
ataagtagag gtttgtagat atgaaaatca gggaatgttt tcttgtagtt caatgtttgg 1500
ctgacacctg ccttttgtta tctctggaat actctgttaa gttcttaagt aattatagct 1560
aatgttgtag ttttattatt cattacatag ccattttaat tattggttaa tttcatcttg 1620
gtctattggc gatagaataa tagtcgggtc ttgccaattt cgttgtcatg agaaaatagg 1680
accactttta gcagagagtt gttattcaca cccctgtttt tctaagacat ttccctaacg 1740
agttctttca aaatttgccc aaaatttcct gtatggaagt accttctggg gaatatattc 1800
ccatttctta cttattatac cttctgaaag tgtgctttca gagtatttat aactttcaga 1860
caattctagt gcatatcttt tatacatttt ggaggttgat tgtgtggatg caatggagta 1920
tttgaagaaa ttagtacatt aatcatttga tagagttttc tgatacatga ttgcattttg 1980
cccctttgct agttgcttgg tatacactct aaactctagc agtcccttaa ccttcacgat 2040
ggcccctata atgagtagta taagtgcaaa tgtataatgg gcatatcctc tgatggcatc 2100
ttccaccatt tgttgatcaa agtctagatg tgtgccattc acgatggccc ctatttgaat 2160
tttatcttta tatttattgc agaagaaaac acattgttat atatttacca aaaatattta 2220
tttaatagta aaaaatttac gaaaaccaag aaccagtctt tgtgatggca atttctttta 2280
ttttatggaa tagcaaatcc atatcttgat gcgcacgcgc gtattataaa caataataat 2340
actcttttta tttttcaaaa aaaatttttt tttagagcaa acaataataa tactttacaa 2400
tttctttttc cttaaaaaag caataatact ttacaaaaaa aaaaaaaaaa aacactcttg 2460
ttttccgcta aaaaaataac actttttacg agaacaaaac tatatgtaat ttcttctcta 2520
agaaaaacta tatatggttt ttctcctaaa aaaactatat ataatatctg taatagaaaa 2580
agctatatat ttcaaagaaa atcaaacaaa tatactctgt ggaatggaat gatgaattaa 2640
ggggttgtta gacaataatc gagtcaccta aaaattattt tttttatata caaagagaaa 2700
ggaaaaaaaa aacttatcat gatatgcatt ttgttaaaaa taaaaaccaa aattcagttt 2760
tcttagtgtt actcttttgt tttatacttt agccagaaac acatttctaa tttcaatttg 2820
gccaaatgtt gaacagtgaa agttagaata caaatgtagc agttgactct ctctttgtcc 2880
attctaaaac agctcttacc agcctactta atattccctt gtacctatct taatttaatt 2940
catattttat ttcataaaca aatacaataa ctcttatcct tcaaaaataa aaaatacaat 3000
aactcttata ttattatatt tatatgctaa tgtcaatttc aaccatttca ataacgcgtt 3060
ttatatgctc tcattattga acatattata taccatcttg ttctttttct atagttcctt 3120
gtctttgcac attttctaag tcttcaaaaa tattccattt tttcatcacc atg gga 3176
Met Gly
1
tat gga aca aga aaa ctc tca gat gaa tat gaa gtt tca gaa att cta 3224
Tyr Gly Thr Arg Lys Leu Ser Asp Glu Tyr Glu Val Ser Glu Ile Leu
        5                   10                  15
ggt aga ggt gga ttt tct gtt gtt aga aaa ggt aca aaa aaa tca agc 3272

Gly Arg Gly Gly Phe Ser Val Val Arg Lys Gly Thr Lys Lys Ser Ser
20                  25                  30
att gaa gaa gaa aaa tca caa tca caa gta gca atc aaa acc cta aga      3320
Ile Glu Glu Glu Lys Ser Gln Ser Gln Val Ala Ile Lys Thr Leu Arg
35                  40                  45                  50
agg tta ggt gct tca aat aac cct agt gga tta cca aga aaa aaa gat      3368
Arg Leu Gly Ala Ser Asn Asn Pro Ser Gly Leu Pro Arg Lys Lys Asp
                55                  60                  65
att gga gaa aaa agc aca ata ggg ttc cct aca atg aga caa gtt tca      3416
Ile Gly Glu Lys Ser Thr Ile Gly Phe Pro Thr Met Arg Gln Val Ser
            70                  75                  80
gtt tca gat aca tta cta aca aat gag ata ctt gta atg aga cga ata      3464
Val Ser Asp Thr Leu Leu Thr Asn Glu Ile Leu Val Met Arg Arg Ile
        85                  90                  95
gtc gaa aac gtt tcg cca cat cca aat gtg att gat ctt tat gat gta      3512
Val Glu Asn Val Ser Pro His Pro Asn Val Ile Asp Leu Tyr Asp Val
    100                 105                 110
tat gag gac aca aat ggt gtt cat ctt gtt ctt gag ctt tgt tcc ggt      3560
Tyr Glu Asp Thr Asn Gly Val His Leu Val Leu Glu Leu Cys Ser Gly
115                 120                 125                 130
ggt gaa ctt ttc gat agg att gtt gca caa gat aag tat agt gag act      3608
Gly Glu Leu Phe Asp Arg Ile Val Ala Gln Asp Lys Tyr Ser Glu Thr
                135                 140                 145
gaa gct gca act gtg gtt cat caa ata gct tca ggg tta gaa gct gtt      3656
Glu Ala Ala Thr Val Val His Gln Ile Ala Ser Gly Leu Glu Ala Val
            150                 155                 160
cat aga gct aat ata gtt cat aga gat ttg aaa cct gaa aat tgt ctt      3704
His Arg Ala Asn Ile Val His Arg Asp Leu Lys Pro Glu Asn Cys Leu
        165                 170                 175
ttt tta gat gtt agg aaa gat tct cct ctt aag att atg gat ttt ggg      3752
Phe Leu Asp Val Arg Lys Asp Ser Pro Leu Lys Ile Met Asp Phe Gly
    180                 185                 190
ttg agt tct gtt gaa gag ttt act gat cct gtt gtt ggt ttg ttt gga      3800
Leu Ser Ser Val Glu Glu Phe Thr Asp Pro Val Val Gly Leu Phe Gly
195                 200                 205                 210
tct att gat tat gtt tca cct gag gct ctt tct caa gga aag att act      3848
Ser Ile Asp Tyr Val Ser Pro Glu Ala Leu Ser Gln Gly Lys Ile Thr
                215                 220                 225

act aag agt gat atg tgg tct ctt ggg gtt att cta tat atc tta ctt     3896
Thr Lys Ser Asp Met Trp Ser Leu Gly Val Ile Leu Tyr Ile Leu Leu
            230                 235                 240
tca gg  gtatgttttt acttttcttt ctttcttata tttaaattgt attaggtttt     3951
Ser Gly

atgttttgat tctagcgatg atgagtttgt atgtatcatc gtggttttaa caaaactcca   4011
ctttagatct tctacaaaat tacggtgcta ctgtgatttc gtcaaactca acatgtccaa   4071
acatgtacta gtagtatgtt tagttttaaa tttggaattt acaaacctaa cctacaagaa   4131
atagcgccga tttgggaaca cggtagctca catctcaccg cgtggtagct cgtctctcca   4191
aattaatagt gcgtttggta ttatgtttgg aatttccaac ccaaacctac aagaaatagt   4251
gtctgtttgg aaacatgtag cttacctctc acagcgaggt agctcattac ttcaagatga   4311
acttcctcga agtgacatca taaaaaaaga aacccaaaca aacacatgct taagcttttg   4371
ttagatacct tgtgatttcg ttaaactcaa cgtatccaaa catatactag tggtatgttt   4431
agttttaaaa ttattttcat ataattgtta tgcag g tat cca cct ttc att gcc   4485
                                        Tyr Pro Pro Phe Ile Ala
                                        245                 250
caa aat aat cgc caa aaa caa caa atg ata atg aat gtaagtacca         4531
Gln Asn Asn Arg Gln Lys Gln Gln Met Ile Met Asn
                255                 260
tctcttacag aatatagaag ttctttccac aattactttg cttactaaaa caaaatggtt   4591
tggaacag ggg aat ttt agt ttt tat gag aag act tgg aag gga att tca   4641
         Gly Asn Phe Ser Phe Tyr Glu Lys Thr Trp Lys Gly Ile Ser
                 265                 270                 275
caa cca gca aag aat ttg att tca agt ctt tta acc gtt gat cct agc     4689
Gln Pro Ala Lys Asn Leu Ile Ser Ser Leu Leu Thr Val Asp Pro Ser
            280                 285                 290
aag aga cct agt gct ctt gag gtattgtaca ctactactat cttgaacata       4740
Lys Arg Pro Ser Ala Leu Glu
        295
ccaacttaac attaaatgtc cacataaata ttttttaatc gagtttgatg aaattagaat    4800
aatgatgtag atattttatg acttaaagga ccaatttata acaaaaaaaa cttaaatgac    4860
taacttgcta taatacaact taagaatcgt agatgatatt tgacctattt ttcatgacat   4920
tatcttcaaa tgtaaagaag tttaaggtga ataaaaacca caacacacat gttgttgtac   4980
tttgtgtttt ttggaacag ctt cta agt gat cca tgg gtc aaa ggt gag aaa    5032
                    Leu Leu Ser Asp Pro Trp Val Lys Gly Glu Lys
                    300                 305                 310
gcc aaa gat gtt caa atg gac cct gag att gtc tca agg cta caa agc     5080

Ala Lys Asp Val Gln Met Asp Pro Glu Ile Val Ser Arg Leu Gln Ser
                315                 320                 325
ttt aat gca aga cgt aaa ctt cgt gca gct gca att gct agt gtt tgg 5128
Phe Asn Ala Arg Arg Lys Leu Arg Ala Ala Ala Ile Ala Ser Val Trp
            330                 335                 340
agc tcc aca atc ttc ctt aga aca aaa aaa ttg aaa tca ttg gtt gga 5176
Ser Ser Thr Ile Phe Leu Arg Thr Lys Lys Leu Lys Ser Leu Val Gly
        345                 350                 355
tcc tat gat ctt aaa gaa gag gaa att gaa aat ctc agg atg cat ttc 5224
Ser Tyr Asp Leu Lys Glu Glu Glu Ile Glu Asn Leu Arg Met His Phe
    360                 365                 370
aag aag at gtaagtaccc aaaagtcaaa aattatcata gtaacattta 5272
Lys Lys Ile
375
actttagctc ttaaatttca caaggaacca aacattttaa gtaaataatc aaattagtta 5332
agaatgacag ataattaaaa tattcaaaag tattcagaac tcctagctca actggcaaaa 5392
atgtcgaaat tgttaggccg aatgccataa ccggggttct accaaaaaaa aaaatattca 5452
aaagtaattt tcataatatt tacacaattc gagctaaggt tttaaaaaac agttcaaagt 5512
cgcgtttgag atgtgacatc aatgtttttt tatgtctttg tagtaattac aattacattt 5572
gctcgaagtg tattttgaca caaaaaattg ggactatgtg tacgatacac ttcggactaa 5632
tgttttcttt ttgaatacaa agaaaatact aatagaaaat acaccatttc tgatttttca 5692
atattaacaa gtgtgttgca gttgtaaact ttgatagttt tgattaaagt aatgtaatga 5752
gttgttaaca g a tgt gca gat aga gac aat gca act ctg tca gag ttt 5800
Cys Ala Asp Arg Asp Asn Ala Thr Leu Ser Glu Phe
        380                 385
gag gag gtg tta aaa gca atg aat atg tta tca ttg atc cct ttt gct 5848
Glu Glu Val Leu Lys Ala Met Asn Met Leu Ser Leu Ile Pro Phe Ala
390                 395                 400                 405
tct cgt ata ttt gat ttg ttt gac aac aac cgt gat gga aca gtt gac 5896
Ser Arg Ile Phe Asp Leu Phe Asp Asn Asn Arg Asp Gly Thr Val Asp
                410                 415                 420
atg cgt gag ata ctt tgt gga ttt tcc agt ctc aag aat tcc aaa gga 5944
Met Arg Glu Ile Leu Cys Gly Phe Ser Ser Leu Lys Asn Ser Lys Gly
            425                 430                 435
gag gat gct ctt cgt ttg tgc ttc cag gtgaattata ctcaacaccc 5991
Glu Asp Ala Leu Arg Leu Cys Phe Gln
        440                 445
aaacaaatat taagttttac aaatatgaat atgtgaagca aatggattca aattatagat 6051

atttgtgagt tgaaatttat gttgttagta tcagactaga tagttcgact agtttaaact 6111

aaatgttaag gagtttagac attgaattta aacaacacta acgaaatagt aaatattaac 6171

ctaaaattat gtatttaact tagttgatca acattggggc ctcataactt gttagacctg 6231

tacattcaaa ccttagactt agtacaacat gatttatttt atttttctaa gtcttgataa 6291

aacgttcact atatatttag gcttaatttt ttttgtcaag ttttacttac cttcaagctg 6351

aattacggat tattaaagtc tcgttcacct aagaatcgaa gagttaacac caaaataaaa 6411

taaaaattac ttttgacctt taaatatgtt ggactgaatc aataaattag ttttttttta 6471

gaggttgaac caataaatta gtcttttttt taaggaataa attaatgtct ctaaagaaat 6531

agcttaaaaa ttaatgttat gaacatacac tctataacta gttttcaaca tgttaaccac 6591

atttgcctct ttatttttat tttgttggca aatag atg tat gat aca gat aga 6644
Met Tyr Asp Thr Asp Arg
            450

tca ggc tgc atc agc aaa gag gaa gta gca tcc atg ctc agg 6686
Ser Gly Cys Ile Ser Lys Glu Glu Val Ala Ser Met Leu Arg
        455                 460                 465

gtaatgcaca cacatctcaa ttaattagct tcatcttttc actttcatta tattaacaca 6746

catttataca ttaattaaac ag gct ttg cca tat gat tgt ctt cca act gat 6798
Ala Leu Pro Tyr Asp Cys Leu Pro Thr Asp
            470                 475

atc act gaa cct gga aaa ttg gat gag att ttt gac tta atg gat gct 6846
Ile Thr Glu Pro Gly Lys Leu Asp Glu Ile Phe Asp Leu Met Asp Ala
            480                 485                 490

aat aat gat gga aaa gtt aca ttt gat gaa ttc aaa gct gct atg caa 6894
Asn Asn Asp Gly Lys Val Thr Phe Asp Glu Phe Lys Ala Ala Met Gln
        495                 500                 505

aga gat agc tct ctt caa gat gta gtt ctc tct tct att cgt cca taa 6942
Arg Asp Ser Ser Leu Gln Asp Val Val Leu Ser Ser Ile Arg Pro
    510                 515                 520

tagttttatg ttcatgatgt tatggtattt taatacttga gtacttttat gctcctttga 7002

ggactttttt atgaagtttt cgtagtaaaa catagtgttc aggcccgacc ctgatatttt 7062

tggtgccctt atcgagtaaa ataatcatat cattcaacaa cttatcgagt attccaccat 7122

caatgtgaag tgttggtcaa ttcaacaact ctcgtgaatc gtttgctgtc tcttctaggg 7182

tttcactgtt cgccgtctac ccctgtgagt gtgatcgtcg tttgtttagg ttctcataat 7242

ttgtatgttt tggatcttgc ctaatctatg tatcatgtat gattatggtg atcaagtatg 7302

catgcgtatg tggtaattgg attagatttc aattgatggg tcttacatta gatttgttgc 7362

atagataata gtttttttat ctctcccaca aaaatcctca agcgtgtctc tatttcaagc 7422

tactcgatct ggttatatgt gttggtatga tctggattcc atggacggtt tcaagacccg 7482

gcgagcccgg gtagtcgccc aagctctggc ccaaattttt tgcaatttct tttgtaaatc 7542

tccttgaatt tcttatataa acccctataa attgggtaat ttctctatct agtattttat 7602
tatttgcttg caatttttta tataaacccc tataaatgtg ttgaattttt tttttgccaa 7662
ggctttataa taatcctgga tccgcatact atttatcatc ccctagtaga gagtcgaatt 7722
aagcttggca tatggttacc atgtgttctt atcaaacatt agtctataaa aaatagtatt 7782
ataagttgga attaccaagg cttaggaaac ctgagtgtat agcttcaaaa ttgaaataca 7842
tagtccgtta ctataagcca aatgctttat ttctaaatga actttagtcc actccaataa 7902
aactgaagaa ttacgttgtt tgcttgattt tgatagttgt ttagcaatta gcagtcttcg 7962
tctaaatggt ggcttagcgc tatcttggag caactctttt aattgtattg tgttaaatta 8022
tatgcaaatc atattaatgt agaagttcat gatcctcgac aaggtaaatg acaattcaca 8082
agtttttaca acttcttgga agagggagaa ggtgtgcctc atgggatttc ttgcataata 8142
tgtcacaaag tatttcgctc ccatggtgta ttatgggtga ctttattgat atcatgggtg 8202
tccgtgagaa acggggtggc tatgaacgtt ctatgtggtt gataagcggt ttcaggcaac 8262
catggctcag tagtggatcc ctcccatcta actagccttt ggaaacgaat gacgcatctg 8322
atgatacaag aagacaagta gtggagacag cgcactaaga aacattggta tagagacgat 8382
gatcttaata caaaattttt acatgcctct gccacttctc ggaaaaaggt aatccgcatt 8442
ttttccttag aaaacaacgt tggtgaggga atttcatatg ataatggtat gtgtcaggtt 8502
gcaaaggggt attttgaaga attgttttta ggaagaaaaa tagcattaga gcttcggttg 8562
ttggtgctat tttagaggta gttatagatg aggatagcaa tttccttact actccttttc 8622
atacacaaga gttcaaggaa cccttgtttt ccatgcaccg gacaagtgtc caggaccgaa 8682
tggtttcaat tatgtttttt tttttaaaca ttttttgtct gtttgtagcc ctgatacatt 8742
tcataaatgt tacacatggt tgagtaataa ccaaatccct tcctctctaa actttgataa 8802
cattgccttg attcctcggg ggaatgaaca taagacaatg aaggattggc gaccaattgc 8862
attgtgtata ttctttataa attacatgta aaagttcttg ctaaccgact taagcatata 8922
ttacacaaat gcatctgtga caataagtca ccttttgttt gagagtcttt ttgtaacaat 8982
ttttgttttt tctcatttga tttaactata ctcttttttc tagttctttt ggcacttctg 9042
gtgctataac ttcatgtgtg ttttaatata tttcatttta ccttcttcaa aaataaatat 9102
tttaaaaaat atatataaat tgtttttttt aattattttc ctgaaaacga gaattttacg 9162
tgtaacaaat tttaattact gaaaagataa aaatattttg atttctaatt aaattgaaat 9222
aaaaaaaaaa atcaattttg aatgttatct aaatatgaat catacacaat tgattcttca 9282
tgattcggat gtattaaata tgactaattg agtcatgcat tttttgagtc atacatgttt 9342
ctcgttaaaa acacatctaa ccagtggcgg agccacgtat tggtcagctg gtgcagctgc 9402
atcccctcaa agttttaaat ttagaccaat atttcttatt ttattcattt tgaacctcct 9462
caaattataa ctttgcacca gcttaaatct tgtcactgca caatggtaat aataatagtg 9522
acgtatatgc agctaataat aataggtcaa aacaacgtag ccattgggct ggaccctact 9582
aaatgtagtg atatatgcag ctcataataa taggccaata tatatgttac acttaatact 9642
tgaaaaaaaa ctatgggaca ctcaaaaatt taatttgtat caatatattc ggtctttaaa 9702
ttaatagtta aatgcgttat attttaaaaa aaattacaat aactaaattt attatccaaa 9762
gtatcgacga aataaaattt attgattgtt ttattacgat aaaatataaa aaatttgata 9822

tgattcttat aaacaatgag atgatatttt tttttcttat gaaatgacat tttataaaat 9882
accaatttga aaaataactc tttatttcat aaaaataatt ggttatttta aaaaacacgt 9942
gtaccggtac acttaaaatg taacttgaca tcaatttgga tcaaatttaa gagactaaac 10002
tcttacaatg tgatagacta atgtttaaat ctgacttaaa gagatacttg tgttttgtac 10062
ttgacatatt tcgaaactca aataagatta tcctcaacaa gaagaactta taaaaagaaa 10122
tattaatttg aggataaaaa tgtaaagagg ataaaaatgt gaggtaaata atatttttat 10182
taaaatttag ttattctagt atttatgaat aaaccataat tgtatcttta tcttcataca 10242
tacatatata attataataa tatgtacaaa aaaaataaca ataatgatga taaatttttt 10302
taaaaaaatt gcacctcctg acccgaggtc ctggctacgc cactgcatct aactacttac 10362
aagacaccac caatatgtct tatatcttat aatatacatg tttttctatg cttcatgtat 10422
taaatatgat ttttgaagtc agggttcggc cctttaaatt tggatgtctt ggacgaatca 10482
agagagtggt gtccttataa aaaaaatttc acaaaaattg ataagattaa gagaaatgat 10542
ctaaaagact attaaacatt tcatttgaca ttgtacaaga aaaacacaag tatgaatcat 10602
tgaagtgtga accaattagc tattattcat cttccttttc ctcttctttg atttgttatt 10662
gtttcctata aaaaaaattg accaagcatt taaaactatt taaatatttt cttcgttgaa 10722
ttttttgttg caaaatcatc aataattcgt tcaaaatcaa ggttcttcac aaaaaatcca 10782
tctgtttatt attcactcca tatcattatt tcaatttaag taccccaatt aaactattga 10842
ctatttactt gctttccaca aattactcat cagttcattt gctaattgat tttttggtac 10902
atgaattcaa atattaaaaa agtgtaacaa acacaaattt tttgattacc atacaaaata 10962
tatcatgtaa caaacacgat atgagtatat gtagattttt cctttatcct taataaaagt 11022
gtaacaaact agatcaaata tatgttctta taaatataat caataagaaa aggaaaatta 11082
tcatcgatac atcgagataa aatattttat atacaggcaa accaaaatag gtcgagttga 11142
taaataattt tgttatatgc tttgacaata atttgtagat acatcgacgt acagataa 11200

<210> 2
<211> 523
<212> PRT
<213> Medicago truncatula

<400> 2

Met Gly Tyr Gly Thr Arg Lys Leu Ser Asp Glu Tyr Glu Val Ser Glu
1               5                   10                  15
Ile Leu Gly Arg Gly Gly Phe Ser Val Val Arg Lys Gly Thr Lys Lys
            20                  25                  30
Ser Ser Ile Glu Glu Glu Lys Ser Gln Ser Gln Val Ala Ile Lys Thr
        35                  40                  45

Leu Arg Arg Leu Gly Ala Ser Asn Asn Pro Ser Gly Leu Pro Arg Lys
    50                  55                  60
Lys Asp Ile Gly Glu Lys Ser Thr Ile Gly Phe Pro Thr Met Arg Gln
65                  70                  75                  80
Val Ser Val Ser Asp Thr Leu Leu Thr Asn Glu Ile Leu Val Met Arg
                85                  90                  95
Arg Ile Val Glu Asn Val Ser Pro His Pro Asn Val Ile Asp Leu Tyr
            100                 105                 110
Asp Val Tyr Glu Asp Thr Asn Gly Val His Leu Val Leu Glu Leu Cys
        115                 120                 125
Ser Gly Gly Glu Leu Phe Asp Arg Ile Val Ala Gln Asp Lys Tyr Ser
    130                 135                 140
Glu Thr Glu Ala Ala Thr Val Val His Gln Ile Ala Ser Gly Leu Glu
145                 150                 155                 160
Ala Val His Arg Ala Asn Ile Val His Arg Asp Leu Lys Pro Glu Asn
                165                 170                 175
Cys Leu Phe Leu Asp Val Arg Lys Asp Ser Pro Leu Lys Ile Met Asp
            180                 185                 190
Phe Gly Leu Ser Ser Val Glu Glu Phe Thr Asp Pro Val Val Gly Leu
        195                 200                 205
Phe Gly Ser Ile Asp Tyr Val Ser Pro Glu Ala Leu Ser Gln Gly Lys
    210                 215                 220
Ile Thr Thr Lys Ser Asp Met Trp Ser Leu Gly Val Ile Leu Tyr Ile
225                 230                 235                 240
Leu Leu Ser Gly Tyr Pro Pro Phe Ile Ala Gln Asn Asn Arg Gln Lys
                245                 250                 255
Gln Gln Met Ile Met Asn Gly Asn Phe Ser Phe Tyr Glu Lys Thr Trp
            260                 265                 270
Lys Gly Ile Ser Gln Pro Ala Lys Asn Leu Ile Ser Ser Leu Leu Thr
        275                 280                 285
Val Asp Pro Ser Lys Arg Pro Ser Ala Leu Glu Leu Leu Ser Asp Pro
    290                 295                 300
Trp Val Lys Gly Glu Lys Ala Lys Asp Val Gln Met Asp Pro Glu Ile
305                 310                 315                 320
Val Ser Arg Leu Gln Ser Phe Asn Ala Arg Arg Lys Leu Arg Ala Ala
                325                 330                 335
Ala Ile Ala Ser Val Trp Ser Ser Thr Ile Phe Leu Arg Thr Lys Lys
            340                 345                 350

Leu Lys Ser Leu Val Gly Ser Tyr Asp Leu Lys Glu Glu Glu Ile Glu
        355                 360                 365
Asn Leu Arg Met His Phe Lys Lys Ile Cys Ala Asp Arg Asp Asn Ala
    370                 375                 380
Thr Leu Ser Glu Phe Glu Glu Val Leu Lys Ala Met Asn Met Leu Ser
385                 390                 395                 400
Leu Ile Pro Phe Ala Ser Arg Ile Phe Asp Leu Phe Asp Asn Asn Arg
                405                 410                 415
Asp Gly Thr Val Asp Met Arg Glu Ile Leu Cys Gly Phe Ser Ser Leu
            420                 425                 430
Lys Asn Ser Lys Gly Glu Asp Ala Leu Arg Leu Cys Phe Gln Met Tyr
        435                 440                 445
Asp Thr Asp Arg Ser Gly Cys Ile Ser Lys Glu Glu Val Ala Ser Met
    450                 455                 460
Leu Arg Ala Leu Pro Tyr Asp Cys Leu Pro Thr Asp Ile Thr Glu Pro
465                 470                 475                 480
Gly Lys Leu Asp Glu Ile Phe Asp Leu Met Asp Ala Asn Asn Asp Gly
                485                 490                 495
Lys Val Thr Phe Asp Glu Phe Lys Ala Ala Met Gln Arg Asp Ser Ser
            500                 505                 510
Leu Gln Asp Val Val Leu Ser Ser Ile Arg Pro
        515                 520

<210>  3
<211>  2586
<212>  DNA
<213>  Pisum sativum

<220>
<221>  exon
<222>  (294)..(1048)
<223>

<220>
<221>  exon
<222>  (1219)..(1273)
<223>

<220>
<221> exon
<222> (1349)..(1459)
<223>

<220>
<221> exon
<222> (1541)..(1773)
<223>

<220>
<221> exon
<222> (1978)..(2192)
<223>

<220>
<221> exon
<222> (2284)..(2330)
<223>

<220>
<221> exon
<222> (2416)..(2586)
<223>

<400> 3
cctcttcttt gtccattcta aaacacctct taccaaccta cttaatattc ccttctatct 60
ctcttaattt aattcatatt ttatttcata aacaattcaa ataactctta tagtattatt 120
ataacctata atgtctattt caaccatttc aataacgcgt tttatatctt cattattgaa 180
catattatca ttattacata tcctcttgtt cttttcctct tcatacattt tcttactctt 240
caaaatatat tccatttttt caaactcttt tgtttttgtt aattatcact acc atg 296
Met
1
gaa tat ggt aca aga aaa ctc tca gat gtg tat gaa gtt tca gag att 344
Glu Tyr Gly Thr Arg Lys Leu Ser Asp Val Tyr Glu Val Ser Glu Ile
            5                   10                  15
cta gga aga ggt gga ttt tct gtt gtt aga aaa ggt aca aga aaa tca 392

Leu Gly Arg Gly Gly Phe Ser Val Val Arg Lys Gly Thr Arg Lys Ser
        20                  25                  30
aac aat gat gat gaa aaa tca caa tca caa tca aaa tca caa tca caa 440
Asn Asn Asp Asp Glu Lys Ser Gln Ser Gln Ser Lys Ser Gln Ser Gln
    35                  40                  45
tca caa gta gca atc aaa aca ctt aga aga tta ggt act tca aat aat 488
Ser Gln Val Ala Ile Lys Thr Leu Arg Arg Leu Gly Thr Ser Asn Asn
50                  55                  60                  65
tta cca agg aaa aaa gat ggt ggt gaa aat agc aca gaa act atg atg 536
Leu Pro Arg Lys Lys Asp Gly Gly Glu Asn Ser Thr Glu Thr Met Met
                70                  75                  80
aag ttc cct aca atg aga caa gtt tct gtc tca gat gca tta cta aca 584
Lys Phe Pro Thr Met Arg Gln Val Ser Val Ser Asp Ala Leu Leu Thr
            85                  90                  95
aat gag att ctt gta atg aga aga ata gtc gaa aac gtt tcg ccg cat 632
Asn Glu Ile Leu Val Met Arg Arg Ile Val Glu Asn Val Ser Pro His
        100                 105                 110
ccg aac gtt atc gat ctt tac gat gtt tat gaa gac aca aat ggt gtt 680
Pro Asn Val Ile Asp Leu Tyr Asp Val Tyr Glu Asp Thr Asn Gly Val
    115                 120                 125
cat ctt gtg ctt gag ctt tgt tct ggt ggc gaa ctt ttc gat agg att 728
His Leu Val Leu Glu Leu Cys Ser Gly Gly Glu Leu Phe Asp Arg Ile
130                 135                 140                 145
gtt gca caa gat aag tat agt gag act gaa gct tca act gtg gtt cat 776
Val Ala Gln Asp Lys Tyr Ser Glu Thr Glu Ala Ser Thr Val Val His
                150                 155                 160
cag att gtt gca ggg tta gaa gct att cat aga gct aac att att cat 824
Gln Ile Val Ala Gly Leu Glu Ala Ile His Arg Ala Asn Ile Ile His
            165                 170                 175
agg gat ttg aag cct gaa aat tgt ctt ttt tta gat gtt ggg aaa gat 872
Arg Asp Leu Lys Pro Glu Asn Cys Leu Phe Leu Asp Val Gly Lys Asp
        180                 185                 190
tct agt ctt aag att atg gat ttt ggg ttg agt tct gtt gaa gag ttt 920
Ser Ser Leu Lys Ile Met Asp Phe Gly Leu Ser Ser Val Glu Glu Phe
    195                 200                 205
act gat cct gtt gtt ggt ttg ttt gga tct att gat tat gtt tct cct 968
Thr Asp Pro Val Val Gly Leu Phe Gly Ser Ile Asp Tyr Val Ser Pro
210                 215                 220                 225

gag gct ctt tct caa gga aag att act act aag agt gat atg tgg tct     1016
Glu Ala Leu Ser Gln Gly Lys Ile Thr Thr Lys Ser Asp Met Trp Ser
                230                 235                 240
ctt gga gtc att cta tat atc tta ctt tcc gg  gtatgttttt ttcttttgag   1068
Leu Gly Val Ile Leu Tyr Ile Leu Leu Ser Gly
            245                 250
tatctgtacg agtaacagga gaaactaatt ctttaaacga gagaggacta caatagacga   1128
caaagtattt tctcaagaga tttaacactg ttgtattaag ttggaagaaa tctcttacca   1188
tctcatctaa ttgctttgat tgtttttcag g tat cca cct ttc att gct caa      1240
                                   Tyr Pro Pro Phe Ile Ala Gln
                                               255
aat aat cgc caa aag caa caa atg ata ttg aat gtaagtaaca tcttagacat   1293
Asn Asn Arg Gln Lys Gln Gln Met Ile Leu Asn
260                 265                 270
gataaattca tttcataatg tttttttgtt tactaaataa aatgttttgg aacag gga    1351
                                                             Gly

aat ttt agt ttt tat gag aag act tgg aag gga att tca caa tca gca     1399
Asn Phe Ser Phe Tyr Glu Lys Thr Trp Lys Gly Ile Ser Gln Ser Ala
            275                 280                 285
aag aat ttg att tca agt ctt cta act gtt gat cct gct aaa aga cct     1447
Lys Asn Leu Ile Ser Ser Leu Leu Thr Val Asp Pro Ala Lys Arg Pro
        290                 295                 300
agt gca caa gag gtactctaat tctacactct taataactta agtaattgaa         1499
Ser Ala Gln Glu
    305
aatattgaag tgttttcaat ttttttttgt tattggaaca g ctt tta agt gat cca   1555
                                              Leu Leu Ser Asp Pro
                                                          310
tgg gtt aaa ggt gag aag gcc aaa gat gat caa atg gac cct gaa att     1603
Trp Val Lys Gly Glu Lys Ala Lys Asp Asp Gln Met Asp Pro Glu Ile
        315                 320                 325
gtg tca agg cta caa aga ttc aat gca aga cgc aaa ctt cgc gca gct     1651
Val Ser Arg Leu Gln Arg Phe Asn Ala Arg Arg Lys Leu Arg Ala Ala
    330                 335                 340
gca att gct agt gtt tgg agc tcc aca att ttt cta aga act aaa aaa     1699
Ala Ile Ala Ser Val Trp Ser Ser Thr Ile Phe Leu Arg Thr Lys Lys
345                 350                 355                 360

ttg aaa tcc ttg gtt gga tcc tat gat ctc aaa gaa gat gaa att gaa     1747
Leu Lys Ser Leu Val Gly Ser Tyr Asp Leu Lys Glu Asp Glu Ile Glu
                365                 370                 375
aat ctt agg atg cac ttc aag aaa at  gtaagtacag tattccaaat           1793
Asn Leu Arg Met His Phe Lys Lys Ile
            380
cgcatttgta atgacatgag cgttttttaa atgtctgtgt cattgtcaat gtaatttcta   1853
ttaagtatat ttaacatcaa attttcaata ataagaaaat cgtgaggcgg attgagacta   1913
ctatttaaaa tcttggttcc agctgtgatt ttgatttaac taatggtgct atggttgtta   1973
acag a tgt gca gat aga gac aat gcg act ctt tgt gag ttt gaa gag      2020
       Cys Ala Asp Arg Asp Asn Ala Thr Leu Cys Glu Phe Glu Glu
                       390                 395
gtg tta aaa gca atg aaa atg cca tca ttg atc cct ttc gca gct cgt     2068
Val Leu Lys Ala Met Lys Met Pro Ser Leu Ile Pro Phe Ala Ala Arg
400                 405                 410                 415
ata ttt gat tta ttt gac aac aac cgc gat gga acg gtt gac atg aga     2116
Ile Phe Asp Leu Phe Asp Asn Asn Arg Asp Gly Thr Val Asp Met Arg
                420                 425                 430
gag att ctt tgt ggc ttt tcg agt ctc aag aac tcc aaa gga gag gat     2164
Glu Ile Leu Cys Gly Phe Ser Ser Leu Lys Asn Ser Lys Gly Glu Asp
            435                 440                 445
gct ctc cgt ttg tgt ttt cag gca agg g gtgtgcaaaa atatgattaa         2212
Ala Leu Arg Leu Cys Phe Gln Ala Arg
        450                 455
ttgaaacata tttccaacat gtgaacgcat ttctgatttt atttttgttg gcaattagat   2272
gtatgacaca g at  aga tca gga tgc atc acc aag gaa gaa gta gca tct    2321
             Asp Arg Ser Gly Cys Ile Thr Lys Glu Glu Val Ala Ser
                         460                 465
atg cta aga gtaatgttca ttcctctcaa tgaattagct ttatattttc             2370
Met Leu Arg
470
actgtcatct tttaacacaa gtgtatatat atatttccta aacag gct ttg cca tat   2427
                                                  Ala Leu Pro Tyr
                                                              475
gat tgt ctt cca act gat atc act gaa cct gga aaa ttg gat gag att     2475
Asp Cys Leu Pro Thr Asp Ile Thr Glu Pro Gly Lys Leu Asp Glu Ile
            480                 485                 490
ttt gac tta atg gat gcc aat agt gat gga aaa gtt aca ttt gat gaa     2523

Phe Asp Leu Met Asp Ala Asn Ser Asp Gly Lys Val Thr Phe Asp Glu
        495                 500                 505
ttc aaa gct gca atg caa aga gat agc tct ctt caa gat gta gtt ctc      2571
Phe Lys Ala Ala Met Gln Arg Asp Ser Ser Leu Gln Asp Val Val Leu
    510                 515                 520
tct tct att cgt cca                                                  2586
Ser Ser Ile Arg Pro
525

<210>  4
<211>  529
<212>  PRT
<213>  Pisum sativum

<400>  4

Met Glu Tyr Gly Thr Arg Lys Leu Ser Asp Val Tyr Glu Val Ser Glu
1               5                   10                  15
Ile Leu Gly Arg Gly Gly Phe Ser Val Val Arg Lys Gly Thr Arg Lys
            20                  25                  30
Ser Asn Asn Asp Asp Glu Lys Ser Gln Ser Gln Ser Lys Ser Gln Ser
        35                  40                  45
Gln Ser Gln Val Ala Ile Lys Thr Leu Arg Arg Leu Gly Thr Ser Asn
    50                  55                  60
Asn Leu Pro Arg Lys Lys Asp Gly Gly Glu Asn Ser Thr Glu Thr Met
65                  70                  75                  80
Met Lys Phe Pro Thr Met Arg Gln Val Ser Val Ser Asp Ala Leu Leu
                85                  90                  95
Thr Asn Glu Ile Leu Val Met Arg Arg Ile Val Glu Asn Val Ser Pro
            100                 105                 110
His Pro Asn Val Ile Asp Leu Tyr Asp Val Tyr Glu Asp Thr Asn Gly
        115                 120                 125
Val His Leu Val Leu Glu Leu Cys Ser Gly Gly Glu Leu Phe Asp Arg
    130                 135                 140
Ile Val Ala Gln Asp Lys Tyr Ser Glu Thr Glu Ala Ser Thr Val Val
145                 150                 155                 160
His Gln Ile Val Ala Gly Leu Glu Ala Ile His Arg Ala Asn Ile Ile
                165                 170                 175

His Arg Asp Leu Lys Pro Glu Asn Cys Leu Phe Leu Asp Val Gly Lys
            180                 185                 190
Asp Ser Ser Leu Lys Ile Met Asp Phe Gly Leu Ser Ser Val Glu Glu
        195                 200                 205
Phe Thr Asp Pro Val Val Gly Leu Phe Gly Ser Ile Asp Tyr Val Ser
    210                 215                 220
Pro Glu Ala Leu Ser Gln Gly Lys Ile Thr Thr Lys Ser Asp Met Trp
225                 230                 235                 240
Ser Leu Gly Val Ile Leu Tyr Ile Leu Leu Ser Gly Tyr Pro Pro Phe
                245                 250                 255
Ile Ala Gln Asn Asn Arg Gln Lys Gln Gln Met Ile Leu Asn Gly Asn
            260                 265                 270
Phe Ser Phe Tyr Glu Lys Thr Trp Lys Gly Ile Ser Gln Ser Ala Lys
        275                 280                 285
Asn Leu Ile Ser Ser Leu Leu Thr Val Asp Pro Ala Lys Arg Pro Ser
    290                 295                 300
Ala Gln Glu Leu Leu Ser Asp Pro Trp Val Lys Gly Glu Lys Ala Lys
305                 310                 315                 320
Asp Asp Gln Met Asp Pro Glu Ile Val Ser Arg Leu Gln Arg Phe Asn
                325                 330                 335
Ala Arg Arg Lys Leu Arg Ala Ala Ala Ile Ala Ser Val Trp Ser Ser
            340                 345                 350
Thr Ile Phe Leu Arg Thr Lys Lys Leu Lys Ser Leu Val Gly Ser Tyr
        355                 360                 365
Asp Leu Lys Glu Asp Glu Ile Glu Asn Leu Arg Met His Phe Lys Lys
    370                 375                 380
Ile Cys Ala Asp Arg Asp Asn Ala Thr Leu Cys Glu Phe Glu Glu Val
385                 390                 395                 400
Leu Lys Ala Met Lys Met Pro Ser Leu Ile Pro Phe Ala Ala Arg Ile
                405                 410                 415
Phe Asp Leu Phe Asp Asn Asn Arg Asp Gly Thr Val Asp Met Arg Glu
            420                 425                 430
Ile Leu Cys Gly Phe Ser Ser Leu Lys Asn Ser Lys Gly Glu Asp Ala
        435                 440                 445
Leu Arg Leu Cys Phe Gln Ala Arg Asp Arg Ser Gly Cys Ile Thr Lys
    450                 455                 460
Glu Glu Val Ala Ser Met Leu Arg Ala Leu Pro Tyr Asp Cys Leu Pro
465                 470                 475                 480

Thr Asp Ile Thr Glu Pro Gly Lys Leu Asp Glu Ile Phe Asp Leu Met
                485                 490                 495
Asp Ala Asn Ser Asp Gly Lys Val Thr Phe Asp Glu Phe Lys Ala Ala
            500                 505                 510
Met Gln Arg Asp Ser Ser Leu Gln Asp Val Val Leu Ser Ser Ile Arg
        515                 520                 525
Pro

<210>  5
<211>  20
<212>  DNA
<213>  Artificial sequence

<220>
<223>  PCR primer

<400>  5
ggccaaatgt tgaacagtga                                                   20

<210>  6
<211>  22
<212>  DNA
<213>  Artificial sequence

<220>
<223>  PCR primer

<400>  6
gggttttgat tgctacttgt ga                                                22

<210>  7
<211>  20
<212>  DNA
<213>  Artificial sequence

<220>
<223>  PCR primer

<400>  7
tcaccatggg atatggaaca                                                    20

<210>  8
<211>  20
<212>  DNA
<213>  Artificial sequence

<220>
<223>  PCR primer

<400>  8
ccacagttgc agcttcagtc                                                    20

<210>  9
<211>  20
<212>  DNA
<213>  Artificial sequence

<220>
<223>  PCR primer

<400>  9
ctttgttccg gtggtgaact                                                    20

<210>  10
<211>  21
<212>  DNA
<213>  Artificial sequence

<220>
<223>  PCR primer

<400>  10
tggacatgtt gagtttgacg a                                                  21

<210>  11
<211>  22

<212>  DNA
<213>  Artificial sequence

<220>
<223>  PCR primer

<400>  11
tcgtggtttt aacaaaactc ca                                                22

<210>  12
<211>  20
<212>  DNA
<213>  Artificial sequence

<220>
<223>  PCR primer

<400>  12
attttgggca atgaaaggtg                                                   20

<210>  13
<211>  24
<212>  DNA
<213>  Artificial sequence

<220>
<223>  PCR primer

<400>  13
tttcgttaaa ctcaacgtat ccaa                                              24

<210>  14
<211>  25
<212>  DNA
<213>  Artificial sequence

<220>
<223>  PCR primer

<400>  14
catttgaaga taatgtcatg aaaaa                                          25

<210>  15
<211>  26
<212>  DNA
<213>  Artificial sequence

<220>
<223>  PCR primer

<400>  15
tgacttaaag gaccaattta taacaa                                         26

<210>  16
<211>  20
<212>  DNA
<213>  Artificial sequence

<220>
<223>  PCR primer

<400>  16
tcgacatttt tgccagttga                                                20

<210>  17
<211>  22
<212>  DNA
<213>  Artificial sequence

<220>
<223>  PCR primer

<400>  17
aaatttcaca aggaaccaaa ca                                             22

<210>  18
<211>  23

<212>  DNA
<213>  Artificial sequence

<220>
<223>  PCR primer

<400>  18
gaagcaaaag ggatcaatga taa                                              23

<210>  19
<211>  22
<212>  DNA
<213>  Artificial sequence

<220>
<223>  PCR primer

<400>  19
tgtgcagata gagacaatgc aa                                               22

<210>  20
<211>  20
<212>  DNA
<213>  Artificial sequence

<220>
<223>  PCR primer

<400>  20
caagttatga ggccccaatg                                                  20

<210>  21
<211>  20
<212>  DNA
<213>  Artificial sequence

<220>
<223>  PCR primer

<400>  21
caacattggg gcctcataac                                                    20

<210>  22
<211>  21
<212>  DNA
<213>  Artificial sequence

<220>
<223>  PCR primer

<400>  22
caatcatatg gcaaagcctg t                                                  21

<210>  23
<211>  20
<212>  DNA
<213>  Artificial sequence

<220>
<223>  PCR primer

<400>  23
atccatgctc agggtaatgc                                                    20

<210>  24
<211>  20
<212>  DNA
<213>  Artificial sequence

<220>
<223>  PCR primer

<400>  24
acgacgatca cactcacagg                                                    20

<210> 25
<211> 24
<212> DNA
<213> Artificial sequence

<220>

<223> PCR primer
<400> 25
tcattgatcc cttttgcttc tcgt 24

<210> 26
<211> 25
<212> DNA
<213> Artificial sequence

<220>
<223> PCR primer

<400> 26
gatgctactt cctctttgct gatgc 25

<210> 27
<211> 26
<212> DNA
<213> Artificial sequence

<220>
<223> PCR primer

<400> 27
tggcatcact cagtaccttt caacag 26

<210> 28
<211> 28
<212> DNA
<213> Artificial sequence

<220>
<223>  PCR primer

<400>  28
acccaaagca tcaaataata agtcaacc                                          28

<210>  29
<211>  20
<212>  DNA
<213>  Artificial sequence

<220>
<223>  PCR primer

<400>  29
ggtggcgaac ttttcgatag                                                   20

<210>  30
<211>  22
<212>  DNA
<213>  Artificial sequence

<220>
<223>  PCR primer

<400>  30
acctgaaaaa caatcaaagc aa                                                22

<210>  31
<211>  22
<212>  DNA
<213>  Artificial sequence

<220>
<223>  PCR primer

<400>  31
gcagttgact ctctctttgt cc                                                22

<210> 32
<211> 21
<212> DNA
<213> Artificial sequence

<220>
<223> PCR primer

<400> 32
tctgtgctat tttcaccacc a 21

<210> 33
<211> 25
<212> DNA
<213> Artificial sequence

<220>
<223> PCR primer

<400> 33
aacgagagag gactacaata gacga 25

<210> 34
<211> 20
<212> DNA
<213> Artificial sequence

<220>
<223> PCR primer

<400> 34
tcaaaatcac agctggaacc 20

<210> 35
<211> 22
<212> DNA
<213> Artificial sequence

<220>
<223>  PCR primer

<400>  35
ggaagaggtg gattttctgt tg                                              22

<210>  36
<211>  21
<212>  DNA
<213>  Artificial sequence

<220>
<223>  PCR primer

<400>  36
tcaggcttca aatccctatg a                                               21

<210>  37
<211>  22
<212>  DNA
<213>  Artificial sequence

<220>
<223>  PCR primer

<400>  37
aaaattgaaa tccttggttg ga                                              22

<210>  38
<211>  20
<212>  DNA
<213>  Artificial sequence

<220>
<223>  PCR primer

<400>  38
aacacaaacg gagagcatcc                                                 20

<210> 39
<211> 20
<212> DNA
<213> Artificial sequence

<220>
<223> PCR primer

<400> 39
agaaaatcgt gaggcggatt 20

<210> 40
<211> 20
<212> DNA
<213> Artificial sequence

<220>

<223> PCR primer
<400> 40
ttggtgatgc atcctgatct 20

<210> 41
<211> 20
<212> DNA
<213> Artificial sequence

<220>
<223> PCR primer

<400> 41
ttgtggcttt tcgagtctca 20

<210> 42
<211> 24
<212> DNA

<213>  Artificial sequence

<220>
<223>  PCR primer

<400>  42
gcagctttga attcatcaaa tgta                                              24

<210>  43
<211>  25
<212>  DNA
<213>  Artificial sequence

<220>
<223>  PCR primer

<400>  43
tggacgaata gaagagagaa ctaca                                             25